# EUROPÄISCHE PATENTSCHRIFT

(11) **EP 1 117 364 B1**
(45) Veröffentlichungstag und Bekanntmachung des Hinweises auf die Patenterteilung: **13.11.2002**
(21) Anmeldenummer: 99932804.0
(22) Anmeldetag: 03.07.1999
(51) Int. Cl.: A61K 6/00

(54) **FLÜSSIGE ZAHNREINIGUNGSMITTEL**
LIQUID TOOTH CLEANING AGENTS
PRODUIT LIQUIDE DESTINE AU NETTOYAGE DES DENTS

(30) Priorität: 14.07.1998 DE 19831547
(43) Veröffentlichungstag der Anmeldung: 25.07.2001
(73) Patentinhaber: Henkel Kommanditgesellschaft auf Aktien, 40589 Düsseldorf (DE)
(72) Erfinder: WÜLKNITZ, Peter, D-42799 Leichlingen (DE); PASTURA, Amerigo, D-58453 Witten (DE); WALTHER-STANGRECKI, Claudia, D-40764 Lagenfeld (DE)
(86) Internationale Anmeldenummer: EP9904631
(87) Internationale Veröffentlichungsnummer: WO00003676

(56) Entgegenhaltungen:
- EP-A- 0 543 442
- DE-A- 4 222 739
- US-A- 5 178 869
- US-A- 5 605 677
- US-A- 5 628 985

## Beschreibung

Die Erfindung betrifft flüssige Zahnreinigungsmittel mit einem Gehalt an Poliermitteln, Feuchthaltemitteln, Bindemitteln und Wasser, die gegenüber herkömmlichen flüssigen Zahnreinigungsmitteln dieser Art eine verbesserte Viskositätskonstanz und ein verbessertes rheologisches Verhalten aufweisen und daher für die Anwendung und Dosierung aus flexiblen Kunststofffläschchen besonders vorteilhaft sind.

Aus dem Stand der Technik, z.B. gemäß DE 42 22 739 A1, EP 543 442 B1 oder US 5,178,869 sind bereits flüssige Zahnreinigungsmittel bekannt, die Poliermittel, z.B. Fällungskieselsäuren, Feuchthaltemittel z.B. Glycerin oder Sorbit und Bindemittel, z.B. Xanthan-Gum enthalten. Ein Problem, das bei solchen Zahnreinigungsmittel besteht, ist die stabile Dispergierung des Poliermittels, das bei Lagerung sich nicht am Boden absetzen darf, ohne daß dabei die Fließfähigkeit verloren geht und die Produkte sich aus Kunststofffläschchen nicht mehr leicht entnehmen und dosieren lassen. Zwar soll die Zubereitung unter der Scherwirkung des eigenen Gewichts im Spendebehälter noch gut fließen, im Ruhezustand, z.B. auf der Zahnbürste soll die Zubereitung aber so dick sein, daß sie nicht oder nur sehr langsam in die Borsten der Zahnbürste einsinken kann.

Während z.B. EP 543 442 B1 einen extrem niedrigen Polyolgehalt vorschlägt, fordert US 5,178,869 eine Fließgrenze von 1 - 60 Pa (20° C), um das rheologische Verhalten im erwünschten Sinne zu beeinflussen. In DE 42 22 739 A1 wird die Viskositätskonstanz durch bestimmte Zusätze von Tensiden, Hydrocolloiden und Polyglycolen erreicht, es wird aber gefordert, daß das Zahnpflegemittel beim Auftragen auf die Zahnbürste zwischen die Borsten einsinkt und eine Viskosität von 2 - 10 Pas aufweist.

Es wurde aber in der Zwischenzeit festgestellt, daß es für die Anwendung solcher Zahnpflegemittel vorteilhaft ist, wenn die Viskosität in einem Bereich von 10 - 100 Pas (20° C) liegt, die thixotrope Zusammensetzung aber bezüglich der unteren und der oberen Fließgrenzen bestimmte Grenzwerte einhält.

Als obere Fließgrenzen (Fₒ) wird dabei der bei der aufsteigenden Messung (mit steigender Scherkraft) der Viskositätskurve erhaltene Extrapolationswert [für D (Schergeschwindigkeit) = 0] und als untere Fließgrenze (Fᵤ) der bei der absteigenden Messung erhaltene Extrapolationswert [für D = 0] der Scherkraft T (Pa) bezeichnet. Dabei wird als Extrapolationshilfe die sog. Casson-Approximation verwendet.

Durch eine Erhöhung des Bindemittelgehalts ließ sich bei sonst konstanter Zusammensetzung zwar die Fließgrenze anheben und das Einsinken der Zusammensetzung in die Borsten der Zahnbürste verlangsamen, gleichzeitig aber stieg die Viskosität bei Lagerung bis zum Verlust der Fließfähigkeit. Eine Senkung des Bindemittelanteils verhinderte zwar das Nachgelieren, senkte aber die Ruheviskosität und die Fließgrenze zu sehr ab, so daß die Paste zu rasch in die Borsten einsinkt. Ähnliche Effekte wurden beim Anheben oder Senken des Poliermittelanteils beobachtet.

Die Erfindung offenbart nun Maßnahmen, mit denen die Einhaltung dieser rheologischen Parameter gelingt. Gegenstand der Erfindung sind daher flüssige Zahnreinigungsmittel mit einem Gehalt an Poliermitteln, Feuchthaltemitteln, Bindemitteln und Wasser, die

| | |
|---|---|
| 5 - 15 Gew.-% | einer Fällungskieselsäure mit einer mittleren Teilchengröße von 8 bis 14 µm, einer spezifischen Oberfläche von 40 - 75 m²/g (BET) und einer geringen Verdickungsleistung |
| 20 - 55 Gew.-% | Glycerin, Sorbit oder eines Gemisches davon |
| 0,3 - 1,0 Gew.-% | Xanthan-Gum als Bindemittel |

enthalten und deren Viskosität, gemessen bei 20° C (Brookfield RVT, 4 UpM, Spindel 4 bis 50 Pas, Spindel 5 bis 100 Pas) im Bereich von 10 - 100 Pas liegt und deren untere Fließgrenze (Fᵤ) wenigstens 10 Pa (20° C) und deren obere Fließgrenze (Fₒ) nicht mehr als 2 Fᵤ beträgt (Fᵤ ≥ 10 Pa, Fₒ ≤ 2 Fᵤ). Durch diese Fließgrenzen wird erreicht, daß die Paste unter dem Einfluß einer Scherkraft oberhalb 10 Pa, z.B. beim Drücken auf den Spendebehälter, zu fließen beginnt, und nach dem Austritt aus der Öffnung, d.h. auf der Zahnbürste, im Ruhezustand relativ hochviskos erscheint.

Es zeigte sich, daß die Qualität der Fällungskieselsäure, die als Polierkomponente enthalten ist, einen sehr entscheidenden Einfluß auf die Rheologie hat. Es genügt z.B. nicht, ein Handelsprodukt wie z.B. Sident 12 einzusetzen, das eine spezifische Oberfläche von 80 m²/g (nach BET) und eine mittlere Korngröße von 6 µm aufweist, da eine solche Kieselsäure immer noch eine beträchtliche Verdickungsleistung im wäßrigen System aufweist. Erfindungsgemäß muß die mittlere Teilchengröße im Bereich von 8 - 14 µm liegen und die spezifische Oberfläche bei 40 - 75 m²/g (nach BET).

Die Verdickungsleistung einer Standard-Dispersion aus 20 Gew.-% Kieselsäure, 40 Gew.-% Glycerin, 37 Gew.-% Wasser, 2 Gew.-% Polyethylenglycol 400 und 1 Gew.-% Carboxymethylcellulose sollte unter 5 Pas (20° C, Brookfield, RVT Spindel 1 und 5) liegen. Der entsprechende Wert für Sident®12 beträgt 15,5 Pas. Als handelsübliche Kieselsäuren mit den erfindungsgemäßen Merkmalen sind Sident®8 (Degussa) und Sorbosil®AC 39 (Crosfield Chemicals) sowie ein Produkt der Bezeichnung RP-LA 2981 (Rhône Poulenc) geeignet. Die Verdickungsleistung beträgt für Sident®8 ca. 1,8 Pas und für Sorbosil® AC39 ca. 1,6 Pas (20° C, Brookfield, RVT, Spindel 1 bzw. 5).

Die erfindungsgemäßen Zahnreinigungsmittel können neben den genannten, nicht verdickenden Fällungskieselsäuren auch noch weitere Polierkomponenten in Mengen von bis zu 5 Gew.-% enthalten. Solche Komponenten könnten z.B. Aluminiumoxide, Dicalciumphosphat-dihydrat, Calciumcarbonat, Calciumpyrophosphat oder andere Polierkomponenten sein, die selbst keine nennenswerten Verdickungseigenschaften haben. Verdickende Kieselsäuren sollten nicht oder nur in Mengen unter 1 Gew.-% enthalten sein.

Als Feuchthaltemittel ist erfindungsgemäß Glycerin, Sorbit oder ein Gemisch aus Glycerin und Sorbit in einer Menge von 20 - 55 Gew.-% enthalten. Dabei ist Glycerin und Sorbit bevorzugt in einem Gewichtsverhältnis von 6 : 4 bis 4 : 6 enthalten. Daneben können in untergeordneten Mengen von bis zu 5 Gew.-% auch andere bekannte Diole oder Polyole oder Polyethylenglycole enthalten sein. Solche anderen Feuchthaltemittel sind z.B. 1,2-Propylenglycol, Xylit und Polyethylenglycole mit Molgewichten von 200 bis 10 000.

Das Bindemittel besteht vorzugsweise ausschließlich aus Xanthan-Gum, es können aber auch andere Bindemittel zusätzlich in Mengen von bis zu 0,5 Gew.-% enthalten sein. Solche zusätzlichen Bindemittel sind z.B. Carragheen, Traganth, Guar, Stärke, Stärke- und Celluloseether, Agar-Agar, Pectine, Polyvinylalkohol, Polyvinylpyrrolidon und Polyethylenglycole mit Molekulargewichten von mehr als 10 000.

Darüber hinaus können die erfindungsgemäßen Zahnreinigungsmitteln übliche Hilfsmittel und Zusatzstoffe in Mengen bis zu 10 Gew.-% enthalten.

Die erfindungsgemäßen flüssigen Zahnreinigungsmittel können z.B. durch Zusatz von Aromaölen und Süßungsmitteln in ihren organoleptischen Eigenschaften verbessert werden. Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Maltose, Fructose.

Weitere übliche Hilfs- und Zusatzstoffe für Zahnpasten sind
- Konservierungsmittel und antimikrobielle Stoffe, z.B. p-Hydroxybenzoesäuremethyl-, -ethyl- oder -propylester, Natriumsorbat, Natriumbenzoat, Bromchlorophen, Triclosan, Phenyl-salicylsäureester, Biguanide, z.B. Chlorhexidin und Peroxide
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Fluorverbindungen wie z.B. Na-Fluorid, Zink-fluorid, Natrium-monofluorophosphat, Aminfluorid u.a.
- Pigmente, wie z.B. Titandioxid
- Farbstoffe
- Puffersubstanzen, z.B. primäre, sekundäre oder tertiäre Alkaliphosphate oder Citronensäure-/Na-Citrat
- wundheilende oder entzündungshemmende Stoffe, z.B. Allantoin, Harnstoff, Azulen, Kamillewirkstoffe. Acetylsalicylsäurederivate oder Rhodanid
- Vitamine wie z.B. Ascorbinsäure, Retinol oder Tocopherol
- Mineralsalze wie z.B. Mangan-, Zink- oder Magnesiumsalze.

Die folgenden Beispiele sollen den Erfindungsgegenstand näher erläutern:

| **Beispiele** | | | | | | | |
|---|---|---|---|---|---|---|---|
| | 1 | 2 | 3 | 4 | 5 | 6 | V |
| Sident®8 | 12 | 12 | 12 | 12 | 12 | 12 | - |
| Sident®12 | - | - | - | - | - | - | 12 |
| Glycerin | 28 | 28 | 28 | 28 | 28 | 28 | 28 |
| Sorbit | 21 | 21 | 21 | 21 | 21 | 21 | 21 |
| Keltrol® F | 0,36 | 0,4 | 0,45 | 0,6 | 0,8 | 1,0 | 0,26 |
| Hydroxypropyl-Guar | 0,04 | - | - | - | - | - | - |
| Na-Fluorid | 0,33 | 0,33 | 0,33 | 0,33 | 0,33 | 0,33 | 0,33 |
| Na-Saccharinat | 0,2 | 0,2 | 0,2 | 0,2 | 0,2 | 0,2 | 0,2 |
| Na-Laurylsulfat | 1,44 | 1,44 | 1,44 | 1,44 | 1,44 | 1,44 | 1,44 |
| Tagat®S | 0,5 | 0,5 | 0,5 | 0,5 | 0,5 | 0,5 | 0,5 |
| Aroma | 1,0 | 1,0 | 1,0 | 1,0 | 1,0 | 1,0 | 1,0 |
| Ethanol | 2,0 | 2,0 | 2,0 | 2,0 | 2,0 | 2,0 | 2,0 |
| Wasser | ad 100 | ad 100 | ad 100 | ad 100 | ad 100 | ad 100 | ad 100 |
| Viskosität¹ (Pas) | 22 | 23 | 24 | 50 | 63 | 94 | 10 |
| Fₒ (nach 10 Tagen) [Pa] | 24 | 21 | 28 | 35 | 45 | 63 | 30 |
| Fᵤ (nach 10 Tagen) [Pa] | 15 | 14 | 18 | 21 | 30 | 43 | 8 |
| Einsinkzeit (min) | 6,6 | 4,9 | >15 | >15 | >15 | >15 | 1,04 |

| | | | | | | | |
|---|---|---|---|---|---|---|---|
| ¹Die Messung der Viskosität erfolgte mit einem Brookfield-Rotationsviskosimeter, Typ RVT, Spindel 4 bzw. Spindel 5 (für Beispiel 5 und 6) bei 20°C und einer Drehzahl von 4 min⁻¹. | | | | | | | |

Es wurden folgende Handelsprodukte verwendet:
- Sident®8: : Amorphe synthetische Kieselsäure Spez. Oberfläche (n. BET) : 60 m²/g Mittlere Agglomeratteilchengröße: 10 µm Lieferant: DEGUSSA AG
- Sident®12 DS: : Fällungskieselsäure Spez. Oberfläche (n. BET): 80 m²/g Mittlere Agglomeratteilchengröße: 6 µm Lieferant: DEGUSSA AG
- Keltrol®F: : Xanthan-Gum von Fa. KELCO, Brüssel
- Tagat®S: : Polyoxyethylen-(20)-glycerin-monostearat der Fa. Goldschmidt, Essen

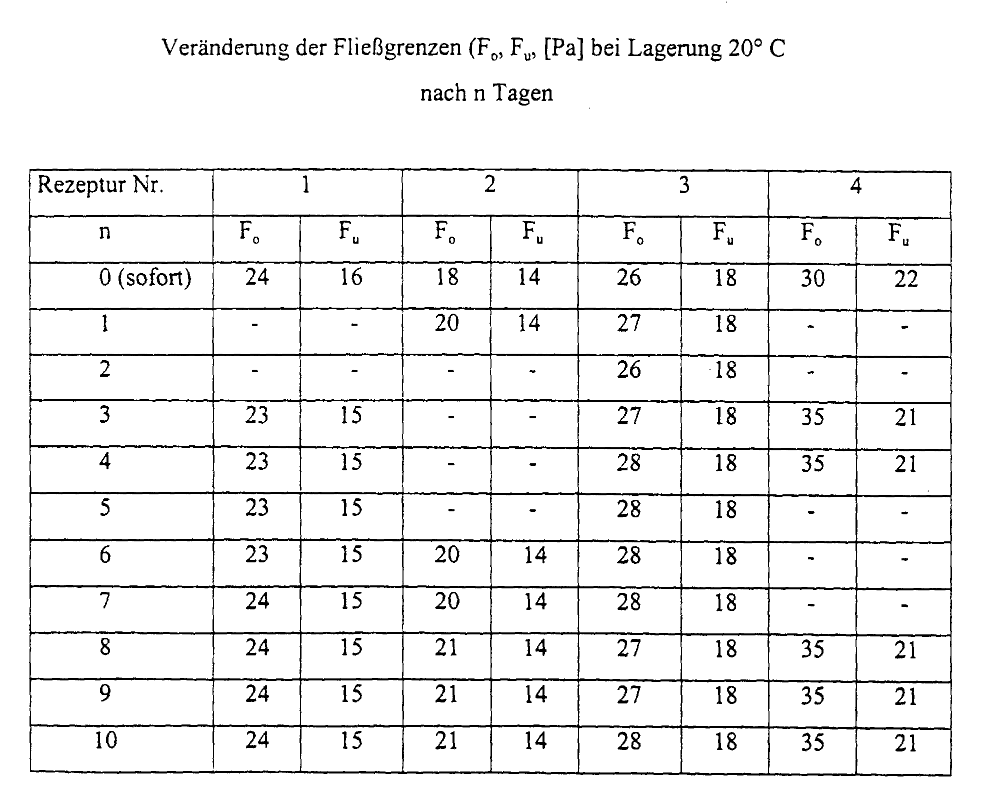

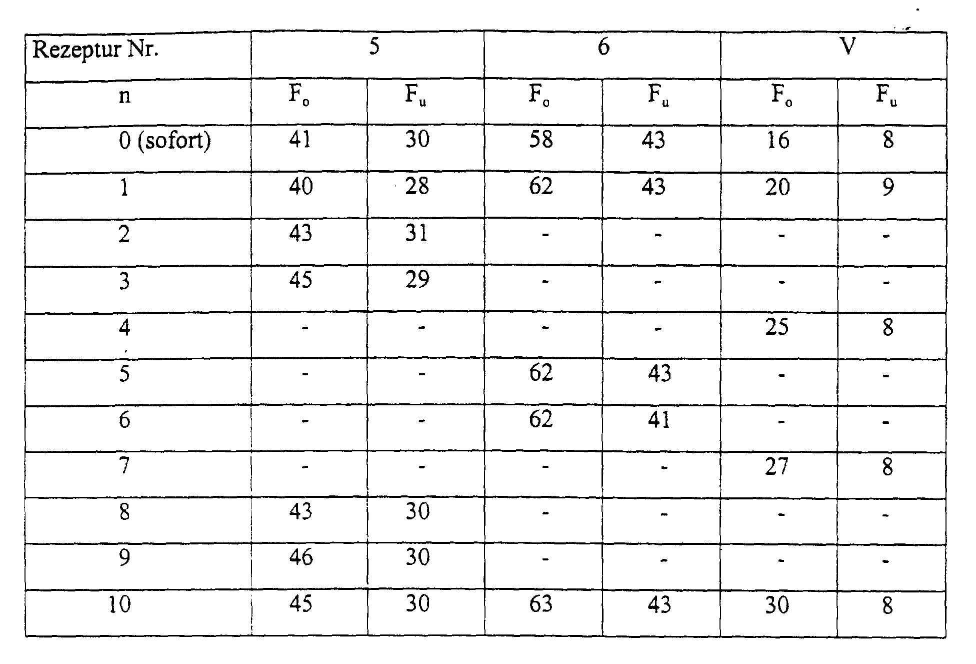

## Patentansprüche

1. Flüssige Zahnreinigungsmittel mit einem Gehalt an Poliermitteln, Feuchthaltemitteln, Bindemitteln und Wasser, **dadurch gekennzeichnet, daß**
| | |
|---|---|
| 5 - 15 Gew.-% | einer Fällungskieselsäure mit einer mittleren Teilchengröße von 8 bis 14 µm, einer spezifischen Oberfläche von 40 - 75 m²/g (BET) und einer geringen Verdickungsleistung |
| 20 - 55 Gew.-% | Glycerin, Sorbit oder eines Gemisches davon |
| 0,3 - 1,0 Gew.-% | Xanthan-Gum als Bindemittel |
enthalten sind und die Viskosität, gemessen bei 20° C (gemessen mit einem Brookfield-Viskosimeter, Typ RVT) im Bereich von 10 - 100 Pas liegt und deren untere Fließgrenze Fᵤ wenigstens 10 Pa (20° C) und die obere Fließgrenze (Fₒ) nicht mehr als 2 Fᵤ beträgt.

2. Flüssige Zahnreinigungsmittel nach Anspruch 1, **dadurch gekennzeichnet, daß** als Feuchthaltemittel ein Gemisch aus Sorbit und Glycerin im Gewichtsverhältnis 6 : 4 bis 4 : 6 enthalten ist.

3. Flüssige Zahnreinigungsmittel gemäß einem der Ansprüche 1 oder 2, **dadurch gekennzeichnet, daß** das Poliermittel ausgewählt ist aus der Gruppe der Handelsprodukte Sident®8 (Degussa) und Sorbosil® AC39.

4. Flüssiges Zahnreinigungsmittel gemäß einem der Ansprüche 1 bis 3, **dadurch gekennzeichnet, daß** sie übliche Hilfs- und Zusatzstoffe aus der Gruppe der Aromen, Süßungsmittel, Konservierungsmittel, oberflächenaktiven Stoffe, Lösungsmittel, Fluorverbindungen, Pigmente, Farbstoffe, Puffersubstanzen, wundheilenden Substanzen, Vitamine und Mineralsalze in Mengen von insgesamt bis zu 10 Gew.-% enthalten.

## Claims

1. Liquid tooth cleaning compositions containing polishing components, humectants, binders and water, **characterized in that** they contain
| | |
|---|---|
| 5 to 15% by weight of | a precipitated silica with a mean particle size of 8 to 14 µm, a specific BET surface of 40 to 75 m²/g and a weak thickening effect, |
| 20 to 55% by weight of | glycerin, sorbitol or a mixture thereof, |
| 0.3 to 1.0% by weight of | xanthan gum as binder, |
and have a viscosity at 20°C (Brookfield RVT viscosimeter) in the range from 10 to 100 Pas, a lower yield point (Yₗ) of at least 10 Pa (20°C) and an upper yield point (Yᵤ) of not more than 2 Yₗ.

2. Liquid tooth cleaning compositions as claimed in claim 1, **characterized in that** a mixture of sorbitol and glycerin in a ratio by weight of 6:4 to 4:6 is present as the humectant.

3. Liquid tooth cleaning compositions as claimed in claim 1 or 2, **characterized in that** the polishing component is selected from the group of commercially available products Sident® 8 (Degussa) and Sorbosil® AC 39.

4. Liquid tooth cleaning compositions as claimed in any of claims 1 to 3, **characterized in that** they contain typical auxiliaries and additives from the group consisting of flavors, sweeteners, preservatives, surfactants, solvents, fluorine compounds, pigments, dyes, buffers, wound-healing substances, vitamins and mineral salts in total quantities of up to 10% by weight.

## Revendications

1. Produits liquides destinés au nettoyage des dents contenant des agents de polissage, des agents d'humidification, des agents liants et de l'eau, **caractérisés en ce qu'**ils renferment
| | |
|---|---|
| 5 à 15 % en poids | d'un acide silicique de précipitation présentant une grosseur de particule moyenne de 8 à 14 µm, une surface spécifique de 40 à 75 m²/g (BET) et un faible pouvoir épaississant |
| 20 à 55 % en poids | de glycérine, de sorbitol ou d'un mélange de ceux-ci |
| 0,3 à 1,0 % en poids | de gomme de xanthane comme liant, |
dont la viscosité, déterminés à 20 °C (viscosimètre Brookfield, type RVT) se situe dans l'intervalle de 10 à 100 Pa.s, et dont la limite de fluidité inférieure (Fᵢ) atteint au moins 10 Pa (à 20 °C) et la limite de fluidité supérieure (Fₛ) n'excède pas 2 Fᵢ

2. Produits liquides destinés au nettoyage des dents selon la revendication 1, **caractérisés en ce qu'**ils contiennent comme agents d'humidification, un mélange de sorbitol et de glycérine dans un rapport ponderal de 6:4 à 4:6.

3. Produits liquides destinés au nettoyage des dents selon une des revendications 1 ou 2, **caractérisés en ce que** l'agent de polissage est sélectionné parmi le groupe des produits commerciaux Siderit®8 (Degussa) et Sorbosil® AC39.

4. Produits liquides destinés au nettoyage des dents selon une des revendications 1 à 3, **caractérisés en qu'**ils contiennent des adjuvants et des additifs usuels appartenant au groupe des arômes, édulcorants, conservateurs, substances tensioactives, solvants, composés fluorés, pigments, colorants, substances tampons, substances vulnéraires, vitamines et sels minéraux, en proportions totales pouvant atteindre 10 % en poids.
